(19) Europäisches Patentamt
European Patent Office
Office européen des brevets

(11) **EP 4 778 502 A1**

(12) **EUROPEAN PATENT APPLICATION**

(43) Date of publication:
**22.07.2026 Bulletin 2026/30**

(21) Application number: **25174504.8**

(22) Date of filing: **06.05.2025**

(51) International Patent Classification (IPC):
**A61H 33/06** (2006.01)

(52) Cooperative Patent Classification (CPC):
**A61H 33/066**; A61H 33/067; A61H 2033/068;
A61H 2201/0188; A61H 2201/10; A61H 2201/102;
A61H 2201/105; A61H 2201/5048

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **21.01.2025 MY PI2025000551**

(71) Applicant: **Tan, Chee Yong
81300 Skudai Johor (MY)**

(72) Inventor: **Tan, Chee Yong
81300 Skudai Johor (MY)**

(74) Representative: **van Dam, Vincent
Octrooibureau Vriesendorp & Gaade B.V.
Koninginnegracht 19
2514 AB Den Haag (NL)**

(54) **WELLNESS ROOM**

(57) The embodiments of the present invention generally pertain to the field of wellness, health therapy, and environmental control systems. Specifically, it pertains to a wellness room (1) designed for installation in residential or commercial spaces, that enhances not only physical wellness but also the user's aura and overall sense of well-being. It is particularly applicable in the areas of heat therapy, detoxification, relaxation, aura enhancement, and overall wellness improvement.

FIG. 1

Processed by Luminess, 75001 PARIS (FR)

EP 4 778 502 A1

**Description**

**TECHNICAL FIELD OF THE INVENTION**

The embodiments of the present invention generally pertain to the field of wellness, health therapy, and environmental control systems. Specifically, it pertains to a modular room, cabin, or chamber designed for installation in residential or commercial spaces, that enhances not only physical wellness but also the user's aura and overall sense of well-being. It is particularly applicable in the areas of heat therapy, detoxification, relaxation, aura enhancement, and overall wellness improvement.

**BACKGROUND OF THE INVENTION**

In today's fast-paced lifestyle, the human body is increasingly impacted by environmental pollution, poor dietary habits, excessive stress, and other detrimental factors. These issues often lead to reduced blood circulation, lower body temperatures, toxin accumulation, and a host of worsening health conditions. To address these challenges and promote overall wellness, sauna rooms and wellness centers have long been conceptualized as solutions.

Traditional sauna rooms and wellness chambers are widely used for their therapeutic and health benefits, primarily by leveraging high-temperature environments to facilitate relaxation and detoxification. Conventional sauna rooms, for example, typically rely on standalone heating methods as part of a traditional heat therapy ritual in a wooden cabin. These saunas maintain an air temperature between 85°C and 100°C, with low humidity to ensure perspiration evaporates effectively. Heat is generated through hot stones heated by a dedicated heater, creating a dry and consistent environment.

However, traditional sauna systems present several limitations. They often lack precise temperature control, are inefficient in creating multi-therapeutic environments, and are not designed for modularity, making them unsuitable for seamless integration into homes. Saunas are generally fixed installations, meaning they cannot be easily disassembled or transported. Moreover, while saunas are considered safe for most people, they are not suitable for individuals with certain health conditions, such as kidney problems, acute infections, inflammation, heart failure, heart disease, or dizziness, further restricting their accessibility.

Other solutions, such as wellness chambers, have attempted to overcome these challenges by incorporating advanced materials like graphene for heat generation. Graphene, known for its excellent thermal conductivity, shows promise in this context. However, its standalone use is limited in versatility, failing to create a truly comprehensive therapeutic environment. Additionally, wellness chambers featuring graphene are often bulky, difficult to install in smaller spaces, and unable to meet the increasing demand for modular systems that adapt to diverse user needs.

Graphene itself, despite its exceptional properties such as antibacterial and moisture-absorbing capabilities, presents certain challenges. It inhibits bacterial growth, prevents odours, and ensures a healthy, clean environment, but its performance in high-temperature or oxygen-rich environments is less stable. When graphene reacts with oxygen at high temperatures, graphene oxide forms, which can degrade its advantageous properties, including its conductivity.

Moreover, heating graphite to produce graphene may result in the release of toxic chemicals. While graphene's antibacterial benefits are well-documented, its standalone and large-scale use with current technology can potentially lead to adverse effects, limiting its application in wellness systems.

The present invention addresses these limitations by introducing a novel solution, particularly a modular, multifunctional wellness or therapy room that seamlessly integrates advanced materials, an intelligent heating system, and additional wellness features. This innovative design offers a versatile and effective therapeutic experience, making it a unique and comprehensive health solution. By combining cutting-edge materials and technology, the invention not only enhances the therapeutic benefits but also provides a more adaptable, accessible, and efficient alternative to traditional sauna rooms and wellness chambers.

**SUMMARY OF THE INVENTION**

The present invention introduces a wellness room, particularly a modular, multifunctional therapy or healthcare room that combines a variety of advanced materials, designed to offer a holistic and customizable therapeutic environment. This invention integrates advanced materials, intelligent heating systems, and modular construction to promote relaxation, detoxification, heat therapy, and even aura enhancement, making it adaptable for health-promoting environment in residential and commercial applications.

Accordingly, the primary aim of the invention is to provide a wellness room that incorporates a combination of advanced materials, including but not limited to graphene, far-infrared emitting substances, antibacterial coatings, high-purity tourmaline integrated with graphene coatings, graphene composite fiber materials, nano-grade graphene films, mangrove plant extracts (such as polyphenols and flavonoids), and brass (a copper-zinc alloy), to create an optimized therapeutic environment. These materials work in synergy to provide uniform heating, enhanced detoxification, and improved energy efficiency, while also ensuring safety and durability.

It is yet another objective of the present invention to provide a modular design for the wellness room, making it easy to install, transport, and integrate into various settings, including homes, offices, and wellness centers. The modularity allows for customizable configurations, ensuring that the wellness room can cater to different space requirements and user preferences.

A further objective of the invention is to incorporate intelligent environmental control systems that allow users to personalize temperature, humidity, and light settings, ensuring a tailored and comfortable wellness experience. The intelligent system also supports features such as air purification and aromatherapy, further enhancing the therapeutic benefits.

Another aim of the invention is to offer a multifunctional environment that goes beyond traditional heat therapy by introducing aura-enhancing elements, such as chromotherapy (colour therapy) and bioenergetic stimulation, to promote mental and emotional well-being alongside physical health.

The present invention also addresses safety and accessibility concerns by using non-toxic, eco-friendly materials and ensuring that the wellness room is suitable for a wide range of users, including those with specific health conditions who may not tolerate traditional saunas.

By achieving these objectives, the present invention provides a comprehensive and advanced solution for promoting health, relaxation, and rejuvenation in a manner that is efficient, customizable, and accessible to a broad audience.

According to the preferred embodiment of the present invention, there is provided,

A wellness room (1), comprising:

> a chamber or room (3) comprising at least three side walls, a base (5e) connected to a roof (5f) via said side walls; at least a heating system (17) incorporating graphene as heating element;

> characterized in that said wellness room (1) further comprising

> > at least a misting mechanism (15) in the chamber or room (3), configured to disperse mist containing therapeutic materials including but not limited to mangrove extract, brass extract, or the combination thereof;

> > wherein the heating system (17) and misting mechanism (15) are arranged to collectively create a wellness-enhancing environment.

## BRIEF DESCRIPTION OF THE DRAWINGS

Other aspect of the present invention and their advantages will be discerned after studying the Detailed Description in conjunction with the accompanying drawings in which:

> FIG. 1 illustrates an exemplary perspective view of a wellness room according to the preferred embodiment of the present invention.

> FIG. 2 illustrates an alternative design example of the wellness room in a different configuration.

## DETAILED DESCRIPTION OF THE DRAWINGS

In the following detailed description, numerous specific details are set forth in order to provide a thorough understanding of the invention. However, it will be understood by the person having ordinary skill in the art that the invention may be practised without these specific details. In other instances, well known methods, procedures and/or components have not been described in detail so as not to obscure the invention.

The invention will be more clearly understood from the following description of the embodiments thereof, given by way of example only with reference to the accompanying drawings, which are not drawn to scale.

As used in this disclosure and the appended claims herein, the singular forms "a", "an", and "the" include plural referents unless the context clearly dictates or denotes otherwise.

Throughout the disclosure and claims of this specification, the word "comprise" and variations of the word, such as "comprising" and "comprises," means "including but not limited to," and is not intended to exclude, for example, other components, integers or steps. "Exemplary" means "an example of" and is not intended to convey an indication of a preferred or ideal embodiment, "such as" is not used in a restrictive sense, but for explanatory purposes.

The term "room," as used throughout this document, is intended to encompass a wide range of structures, including but not limited to cabins, chambers, chalets, cottages, houses, compartments, or similar enclosures. This terminology is deliberately broad to ensure it captures the diverse applications and configurations that can benefit from the innovations described herein.

The "room" in the context of this invention is designed as a modular, transportable, and movable structure capable of being installed in various locations, such as residential houses, commercial buildings, open spaces, or specialized wellness areas. Despite its modular and portable nature, the room is equally adaptable for permanent installation in specific locations, such as wellness centers or designated health therapy spaces, depending on the needs and preferences of the user.

This flexibility ensures that the wellness room can cater to a variety of scenarios, from individual home installations to large-scale, commercial wellness facilities. The design harmoniously integrates the benefits of modularity and mobility with the robustness and functionality required for fixed installations, thereby providing users with an innovative and versatile therapeutic solution.

The term "wellness," as employed throughout this document, is intended to embody a broad spectrum of meanings and applications. It encompasses, but is not limited to, aspects of health, healthcare, physical and mental well-being, relaxation, stress relief, therapeutic practices, rejuvenation, and general self-care. This definition is purposefully expansive to reflect the multifaceted nature of wellness and its significance in promoting a balanced and healthy lifestyle. Whether addressing physical ailments, enhancing mental clarity, fostering relaxation, or supporting holistic therapies, the concept of "wellness" as described herein is designed to cater to diverse needs and preferences. Furthermore, "wellness" also includes innovative approaches to creating environments that prioritize comfort, healing, and the enhancement of overall quality of life. This comprehensive interpretation ensures that the innovations described in this invention align with the evolving demands of modern health and self-care practices, making them suitable for use in personal, professional, and communal settings.

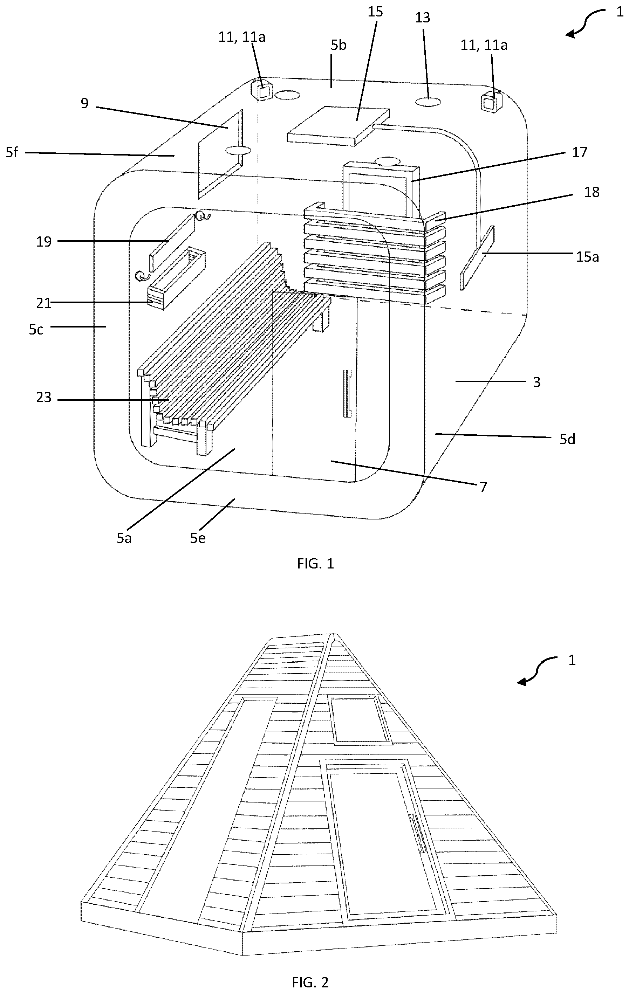

FIG. 1 illustrates an exemplary example of a wellness room (1) according to the preferred embodiment of the present invention, of which is designed or invented for relaxation, rejuvenation, and holistic health enhancement. Said wellness room (1) comprising at least a room or chamber (3) defined by at least three side walls (5a, 5b, 5c). Specifically, the room or chamber (3) comprising at least 4 side walls, namely the front wall (5a), back wall (5b), left wall (5c) and right wall (5d). Said room or chamber (3) further comprising a base (5e) and a roof (5f) connected with the side walls (5a, 5b, 5c, and 5d), creating an enclosed environment optimized for wellness. Said wellness room (1) further comprising at least one door (7) positioned on at least one side wall of the wellness room (1) for user entry and exit, as well as at least one window (9) positioned on at least one side wall of the wellness room (1) to allow for natural light and ventilation, enhancing the overall ambiance.

The wellness room (1) is innovatively engineered to integrate sound therapy, misting mechanisms, and advanced heating systems, working synergistically to create a multi-sensory environment that fosters physical rejuvenation and mental well-being. These components, of which will be explained in detail below, are thoughtfully orchestrated to enhance the user's experience, supporting relaxation, emotional balance, and overall health.

A key feature of the wellness room (1) is its innovative sound therapy system (11), which incorporates at least one high-quality speaker (11a) to deliver immersive audio output. This system utilizes a unique five-tone therapy framework inspired by the principles of the Five Elements theory. By employing specific frequency-based music, the sound therapy aims to resonate harmoniously with the body's internal organs, promoting mental serenity, emotional regulation, and hormonal equilibrium. This holistic approach elevates both the effectiveness and therapeutic value of the wellness room. The sound therapy system (11) is engineered to deliver frequency-specific sound waves that provide a range of targeted therapeutic benefits. These include:

- Low-frequency waves (1-100 Hz): Designed to penetrate deeply into the body, these waves stimulate deep tissue relaxation and enhance nerve function, offering profound physical and mental relaxation.

- Mid-frequency waves (100-2000 Hz): These frequencies are optimized to restore emotional balance and improve sleep quality, addressing stress and emotional dysregulation.

- High-frequency waves (>2000 Hz): Focused on cognitive stimulation, these waves promote mental clarity, emotional uplift, and heightened focus.

Additionally, the sound therapy system (11) incorporates healing frequencies renowned for their therapeutic properties, such as:

- 432 Hz: Known to promote harmonic balance within the body and mind, creating a calming effect.

- 528 Hz: Often referred to as the "DNA repair frequency," this tone is believed to facilitate cellular regeneration and enhance overall vitality.

- 639 Hz: Associated with fostering relationship harmony, this frequency supports emotional connections and promotes a sense of unity.

By combining the ancient wisdom of the Five Elements theory with modern acoustic technology, the sound therapy system (11) in the wellness room (1) delivers an unparalleled therapeutic experience. Its carefully selected range of frequencies and tones works to harmonize the body and mind, ensuring a transformative and rejuvenating journey for every user.

The wellness room (1) further comprising an advanced light therapy system (13), which is equipped with a dynamic seven-colour lighting configuration. This sophisticated system (13) is fully customizable, allowing users to select specific colours to suit their individual therapeutic needs and preferences. Each of the seven colours is carefully chosen for its unique properties, delivering targeted benefits that complement the overall wellness experience. The light therapy system (13) is designed to harness the power of chromotherapy, an ancient practice that uses colours to restore balance and harmony within the body and mind. Each colour is associated with distinct physiological and psychological effects, as follows:

Red Light that stimulates energy, enhances circulation, and promotes vitality, making it ideal for combating fatigue or sluggishness; Orange Light that encourages creativity, boosts mood, and supports emotional well-being, fostering a sense of joy and enthusiasm; Yellow Light that energizes the nervous system, aids digestion, and improves mental clarity, helping users feel focused and alert; Green Light that symbolizes balance and calmness, soothing the mind and promoting emotional stability while reducing stress; Blue Light that induces relaxation, encourages restful sleep, and alleviates anxiety, making it perfect for unwinding after a long day. Indigo Light that enhances intuition, supports spiritual awareness, and stimulates creativity, offering a deeper sense of inner connection. Violet Light that promotes mental clarity, encourages meditation, and fosters a sense of peace, helping users achieve a serene state of mind.

The light therapy system (13) allows users to adjust the intensity and combination of colours, creating personalized lighting environments tailored to their therapeutic goals. Whether seeking relaxation, revitalization, or emotional balance, the seven-colour light therapy system (13) provides a versatile and impactful tool to enhance the multi-sensory experience of the wellness room (1). By integrating modern lighting technology with ancient healing principles, this feature ensures a holistic and transformative journey toward well-being.

The wellness room (1) further comprising an advanced misting mechanism (15) that infuses the environment with a therapeutic mist composed of specialized ingredients. The misting mechanism (15) is designed with mist dispersion means, or precision nozzles or nozzle array strategically located at the ceiling or roof (5f) of the room (3) to ensure uniform dispersion of a fine mist throughout the wellness room. For versatility, the nozzles can be positioned in other areas of the space based on the room's design or user preferences. The present invention utilizes the restorative properties of brass and/or mangrove extracts to deliver a holistic and rejuvenating experience. A dedicated reservoir (15a) stores the brass solution, mangrove extract solution, or the combination thereof, ensuring the bioactive compounds remain pure and effective. Additionally, the misting mechanism (15) includes adjustable mist concentration controls, allowing users to customize the mist's intensity according to their therapeutic needs.

The therapeutic properties of the mist are derived from two key components: brass and mangrove extracts. Brass, an alloy of copper and zinc, offers remarkable antibacterial and immune-enhancing properties. When absorbed through the skin, it provides trace elements that boost energy metabolism, promote cellular repair, and enhance the body's natural defences. Mangrove extracts, particularly from the red mangrove plant, are rich in bioactive compounds such as polyphenols and flavonoids. These compounds deliver significant anti-inflammatory, antibacterial, and antioxidant benefits, supporting skin barrier repair, reducing inflammation, and neutralizing free radicals. Together, these elements create a mist that rejuvenates and protects the skin while promoting overall health.

The synergy between brass and mangrove extracts amplifies the therapeutic impact of the misting mechanism (15). Brass supports immune function and cellular regeneration, while mangrove extracts enhance skin healing and protect against pathogens. This combination also alleviates stress and anxiety, working harmoniously with sound and light therapy system (11, 13) to promote mental tranquility. Additionally, the antioxidant properties of the mist help delay aging, improve skin texture, and restore vitality.

By integrating this misting mechanism, the wellness room (1) offers a unique sensory experience that promotes physical, mental, and emotional well-being. The blend of brass and mangrove extracts exemplifies a forward-thinking approach to holistic health, providing users with an immersive pathway to rejuvenation and balance.

The wellness room (1) further comprising a heating system (17) that utilizes far-infrared technology combined with materials including but is not limited to, graphene, tourmaline, aluminium, alloy, high-performance composites, or the combination thereof. This heating system (17) is designed to provide precise and efficient thermal therapy, promoting overall wellness and relaxation. The heating system (17) leverages graphene, a two-dimensional crystal structure with exceptional thermal conductivity, capable of emitting far-infrared radiation in the 4 to 24-micrometer range. These wavelengths, often referred to as "life light waves," penetrate deeply into the skin, activating cellular activity, boosting metabolism, enhancing blood circulation, and increasing body temperature. The graphene is available in various forms, including coatings, composite fiber materials, and nano-grade films.

To amplify its therapeutic effects, the graphene is integrated with high-purity tourmaline, aluminium, alloy, or the combination thereof, forming a composite material that amplifies the therapeutic effects. Tourmaline contributes its own bioactive properties, including negative ion generation and far-infrared emission, further supporting detoxification and relaxation. Additionally, the composite material may be enriched with essential elements including but not limited to boron, hydrogen, magnesium, iron, lithium, manganese, titanium, carbon, zinc, or brass, or the combinations thereof, further enhancing its therapeutic properties.

The heating system (17) comprises a thermal conduction layer made from high-conductivity materials like graphene composites, tourmaline, copper, aluminum alloys, or the combination thereof. This layer is coated with far-infrared emitting materials serving as a heat release layer to provide uniform heat distribution and deep penetration for therapeutic benefits. The conduction layer's design, including its thickness, shape, and surface treatment, is carefully optimized to achieve varying intensities of heat release.

Heat is generated by a heat source layer that employs electric heating elements, photothermal conversion materials, or chemical heat sources, efficiently transferring thermal energy to the conduction layer. Thermal energy is transferred from the heat source layer to the thermal conduction layer and eventually releases the heat to the heat release layer through the material's thermal conductivity.

The heating system (17) further comprising an intelligent temperature control system to ensure precise regulation of heat output, maintaining a stable temperature range of 40°C to 65°C with an accuracy of $\pm 0.5$°C. The system employs thermistors, thermocouples, and microcontroller-based chips in a closed-loop regulation mechanism to continuously adjust the heat source output, maintaining consistent surface temperatures. The heat released from the heat release layer raises the body's core temperature, supporting processes such as metabolism, detoxification, improved circulation, and the expulsion of toxins like heavy metals and metabolic waste. Far-infrared wavelengths penetrate deeply into the skin, stimulating microcirculation, enhancing oxygen delivery, and supporting cellular repair. This comprehensive process relieves tension and promotes overall health and well-being.

When combined with five-tone therapy and mangrove extract sprays, the heating system (17) creates a holistic environment for physical and mental rejuvenation. This integrated approach not only raises body temperature but also enhances blood circulation and facilitates deep detoxification, significantly improving overall health. The wellness room's uniqueness lies in its fusion of cutting-edge technology and natural elements, achieving synergistic therapeutic effects and representing a revolutionary advancement in health and wellness products.

This state-of-the-art heating mechanism of the present invention is versatile and suitable for various applications, including Heat therapy chambers for relaxation, pain relief, and detoxification; Residential Use like Compact sauna systems for personal wellness routines; and Commercial Use like spas, wellness centers, and healthcare facilities. Its features comprise precise temperature control for enhanced user comfort, efficient heat distribution through high-conductivity materials, energy-efficient operation with advanced insulation to minimize energy loss, and a customizable modular design for flexible installation in diverse environments. The combination of far-infrared radiation and bioactive materials delivers unparalleled therapeutic benefits, making this wellness room (1) an innovative solution for comprehensive health and wellness.

The wellness room (1) further comprises a protective barrier or fence (18) strategically installed to cover the heating system (17). The fence (18) is designed to prevent users from accidentally coming into direct contact with the heating system (17), thereby reducing the risk of burns or injuries. This protective barrier (18) ensures safe use of the wellness room (1) while still allowing for the efficient operation and heat dispersion of the heating system (17).

The wellness room (1) further comprising an intuitive control panel (19) that allows users to manage various features, including lighting modes, sound therapy, temperature settings, mist release concentrations, movable chair positioning. This control panel (19) enables users to personalize their wellness experience by adjusting temperature, humidity, music frequency, and mist concentration to suit their individual preferences. The precise temperature control technology ensures a stable and comfortable thermal environment, eliminating the discomfort often associated with traditional high-temperature saunas while delivering optimal detoxification and metabolic enhancement benefits. Additionally, the control panel (19) offers customizable wellness solutions, allowing users to store and access personalized settings for a truly tailored therapeutic experience.

The wellness room (1) is thoughtfully equipped with dedicated storage compartments (21) designed to enhance convenience and functionality. These storage areas (21) are ideal for keeping essential items such as towels, water bottles, and mobile devices organized and within easy reach, ensuring a clutter-free and comfortable environment.

In addition, the wellness room (1) features a mobile, adjustable chair (23) that elevates the user experience. This ergonomically designed chair (23) provides exceptional comfort and can be easily repositioned to accommodate individual preferences or specific therapeutic needs. Its adjustable settings enable users to customize the seating angle and height, promoting relaxation during various wellness sessions. Together, these features ensure a harmonious blend of practicality and luxury, making the wellness room (1) a comprehensive solution for both relaxation and rejuvenation.
To design the wellness room (1) of the present invention, specific guidelines must be followed to optimize its functionality and effectiveness.

1. Internal Volume

The internal volume of the wellness room (1) plays a critical role in determining airflow and heat flow, forming the foundation for establishing energy parameters. Using advanced formulas, the optimal space volume for the exemplary design is calculated to be 140 cubic meters. This volume ensures proper distribution of air and thermal energy within the room (3), creating a conducive environment for wellness activities. The formula is:

$$a^{1/2}c\xi^- = a^0 \lambda \varepsilon (c\xi^-) \times e^{\langle\lambda a0|}$$

$$a^0 \lambda \varepsilon (c\xi^-) = \varepsilon\lambda \times a\widehat{(r)}^{1/2} = 4a^{13} \times 5a\lambda = 20a^{13}a\varepsilon\lambda^1$$

$$a^{1/2}c\xi^- = 20a^{13}a\varepsilon\lambda^1 \times 7a\lambda = 140c«\lambda a\varepsilon\lambda^1$$

2. Standard Heating Power:

The standard heat power required for the wellness room (1) is determined through precise calculations, accounting for the internal volume and thermal conductivity of the materials used. For the exemplary design, a heating power of approximately 5000 watts is recommended, ensuring efficient and stable thermal energy distribution. It is calculated using the formula:

$$a\lambda c\lambda(a\lambda c!l) = a^{1/2}c\xi^- (c«\lambda a\varepsilon\lambda^1 c\pm^3) \times 1.2$$

$$140c«\lambda a\varepsilon\lambda^1 = 140 \div 35.3 \approx 3.97c«\lambda a\varepsilon\lambda^1 c\pm^3$$

$$a\lambda c\lambda(a\lambda c!l) = 3.97c«\lambda a\varepsilon\lambda^1 c\pm^3 \times 1.2 = 4.76a\lambda c!l$$

3. Airflow and Ventilation

Effective ventilation is achieved by strategically placing ventilation openings at the top and bottom of the wellness room. The vent opening area is calculated as 1-2% of the base area, resulting in a total vent area of approximately 0.2 square meters for the given design. This configuration ensures optimal airflow, maintaining a fresh and comfortable atmosphere within the room. It is calculated using the formula below:

$$\varepsilon\lambda\lambda a\lambda£\varepsilon\lambda(c\xi^-) = 20a^{13}a\varepsilon\lambda^1 \times 1\% = 0.2a^{13}a\varepsilon\lambda^1$$

4. Resonance Frequency Calculation

The resonance frequency, which influences the energy optimization for meditation spaces, is determined by the room's dimensions: the length, width, and height of a room and is an important parameter for optimizing the energy of meditation spaces:

• Length (L) = 5 units, Width (W) = 4 units, Height (H) = 7 units

The formula for calculating the resonance frequency of sound waves (fff) is:

$$f = \frac{v}{2} \sqrt{\left(\frac{n}{L}\right)^2 + \left(\frac{m}{w}\right)^2 + \left(\frac{P}{H}\right)^2}$$

- v=343 m/s (speed of sound in air)
- n, m, p: Vibrational modes (taking the lowest mode where n = m = p = 1).

Substitute values:

$$f = \frac{343}{2} \sqrt{\left(\frac{1}{1.524}\right)^2 + \left(\frac{1}{1.219}\right)^2 + \left(\frac{1}{2.134}\right)^2}$$

f \approx \frac{343}{2} \sqrt{0.431 + 0.672 + 0.22}\ approx \frac{343}{2} \times \sqrt{1.323}

$$f \approx \frac{343}{2} \times 1.15 \approx 197.2 Hz$$

This frequency corresponds to a low-frequency resonance mode, which is suitable for promoting deep relaxation and meditation.

5. Energy Concentration and Light Wave Design

For designs incorporating a far-infrared light therapy device, the recommended wavelength range is 4-24 μm, which aligns with the most beneficial infrared range for the human body. The device should be installed centrally at the top of the wellness room to ensure even energy distribution in all directions. This configuration maximizes the therapeutic benefits of the light waves, supporting overall wellness.

In summary, an exemplary wellness room (1) design would feature a space volume of 140 cubic meters, a heating power of approximately 5000 watts, a vent area of 0.2 square meters, a resonance frequency of 197.2 Hz, and a far-infrared light wave range of 4-24 μm. However, it is acknowledged that alternative parameters may be considered depending on specific applications and requirements, allowing for flexibility and customization in the room's design.

In another embodiment of the present invention, as illustrated in FIG. 2, a wellness room (1) featuring a triangular structure or pyramid configuration is presented. This design takes advantage of the geometric properties of a pyramid to optimize the space for meditation, energy therapy, and spiritual practices. The dimensions and calculations for this structure demonstrate its efficiency and suitability for compact spaces.

Pyramid dimension and calculation

1. Calculating the Diagonal Length (From Base Center to Apex)

Using the Pythagorean theorem to calculate the diagonal surface length (S):

$$s = \sqrt{\left(a/2\right)^2 + H^2}$$

Where:

- a is the base edge length
- H is the height of the pyramid

$$s = \sqrt{\left(\frac{5}{2}\right)^2 + 7^2} = \sqrt{2.5^2 + 7^2} = \sqrt{6.2^5 + 49}$$

$$S = \sqrt{55.25} \approx 7.43 (units)$$

The diagonal surface length, measured from the base center to the apex, is calculated using the Pythagorean theorem. For a base side length of 5 units and a height of 7 units, the diagonal length is approximately 7.43 units.

2. Calculating the Surface Inclination Angle

The inclination angle θ\ theta θ is the angle of the pyramid's slanted surface relative to the ground, given by:

$$\tan(\theta) = \frac{H}{a\,/\,2}$$

$$\tan(\theta) = \frac{7}{2.5} = 2 \cdot 8$$

$$\theta = \tan^{-1}(2.8) \approx 70.02^0$$

The inclination angle of the pyramid's slanted surface relative to the ground is determined using the tangent function, yielding an angle of approximately 70.02°.

These design parameters highlight the precision and intentionality of the structure, with a base measuring 5 units by 5 units, a height of 7 units, a diagonal surface length of 7.43 units, and an inclination angle of 70.02°.

The pyramid configuration offers two key benefits. First, its proportions and sharp angles are believed to enhance energy concentration, making it an ideal space for meditation or energy therapy. Second, the compact design is perfectly suited for smaller spaces dedicated to spiritual cultivation, light stretching, or restful retreats. This embodiment demonstrates an innovative approach to wellness room design, leveraging the unique qualities of pyramid geometry to create a harmonious and effective environment.

The wellness room (1) represents a groundbreaking fusion of advanced material science and traditional therapeutic principles. Its multifunctional design offers state-of-the-art solutions to enhance physical health, emotional well-being, and environmental energy. This invention serves as a transformative tool in the health and wellness industry, with potential applications across both consumer and clinical settings.

The wellness room (1) combines cutting-edge far-infrared technology with tourmaline, graphene, mangrove extracts, brass, five-tone therapy, and customizable thermal experiences to deliver a versatile and highly effective health and wellness solution. This wellness room (1) not only improves physical fitness but also fosters mental and emotional balance, embodying a harmonious blend of modern technology and traditional wisdom.

The wellness industry is experiencing rapid growth worldwide, driven by increasing consumer demand for solutions that merge natural, sustainable, and technological elements. As an innovative product, the wellness room addresses a critical market need, setting a new standard in health and wellness. Its diverse benefits and unique user experiences position it as a highly competitive product with significant growth potential. This innovation not only solidifies market leadership but also introduces a new paradigm in holistic health solutions.

While the present invention has been shown and described herein in what are considered to be the preferred embodiments thereof, illustrating the results and advantages over the prior art obtained through the present invention, the invention is not limited to those specific embodiments. Thus, the forms of the invention shown and described herein are to be taken as illustrative only and other embodiments may be selected without departing from the scope of the present invention, as set forth in the claims appended hereto. The scope of the invention encompasses numerous alternatives, modifications and the equivalents. Of necessity, there are many alternative ways of configuring and implementing the invention to suit particular installations and environments while providing biological outcomes of differing design.

**Claims**

1. A wellness room (1), comprising:

   a chamber or room (3) comprising at least three side walls, a base (5e) connected to a roof (5f) via said side walls;
   at least a heating system (17) incorporating far-infrared heating element;
   **characterized in that** said wellness room (1) further comprising
   at least a misting mechanism (15) in the chamber or room (3), configured to disperse mist containing therapeutic materials including but not limited to mangrove extract, brass extract, or the combination thereof;
   wherein the heating system (17) and misting mechanism (15) are arranged to collectively create a wellness-enhancing environment.

2. The wellness room (1) as claimed in Claim 1, wherein said misting mechanism (15) comprising at least a nozzle or array of nozzles positioned at the roof or other areas in the chamber or room (3); and a reservoir (15a) for storing the therapeutic materials.

3. The wellness room (1) as claimed in Claim 1 or 2, wherein the mangrove extract comprising compounds including but not limited to polyphenols, flavonoids, or the combination thereof.

4. The wellness room (1) as claimed in Claim 1 or 2, wherein said heating system (17) comprising at least a heat source layer for heat generation, at least a thermal conduction layer for conducting heat received from the heat source layer, a heat release layer for releasing heat from the heat conduction layer to the environment in the wellness room (1).

5. The wellness room (1) as claimed in Claim 4, wherein said heat source layer employs electric heating elements, photothermal conversion materials, or chemical heat sources, for transferring thermal energy to the conduction layer; the thermal conduction layer comprising conductivity materials including but not limited to graphene composites, tourmaline, copper, aluminum alloys, boron, hydrogen, magnesium, iron, lithium, manganese, titanium, carbon, zinc, or brass, or the combination thereof; and the heat release layer comprising far-infrared emitting materials.

6. The wellness room (1) as claimed in Claim 4 or 5, wherein the heating system (17) further comprising at least a temperature control system to regulate heat output, maintaining a temperature range of 40°C to 65°C with an accuracy of ±0.5°C.

7. The wellness room (1) as claimed in Claim 1, further comprising a light therapy system (13) configured to emit light with adjustable intensity and a spectrum of colours.

8. The wellness room (1) as claimed in Claim 1 or 7, further comprising sound therapy system (11) configured to emit ambient sounds, music, or therapeutic audio.

9. The wellness room (1) as claimed in 7 or 8, further comprising at least one chair.

10. The wellness room (1) as claimed in 7 or 8, further comprising a control panel (19) configured to manage the operation of the heating system (17), misting system (15), lighting system (13), and sound system (11).

11. The wellness room (1) as claimed in claim 1, further comprising storage compartments (21) integrated into the walls or base of the room for storing items.

12. The wellness room (1) as claimed in claim 1, further comprising at least a door (7) and at least one window (9) to facilitate access and natural lighting.

FIG. 1

FIG. 2

Europäisches
Patentamt

European
Patent Office

Office européen
des brevets

## EUROPEAN SEARCH REPORT

**Application Number**

EP 25 17 4504

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/294819 A1 (LEVESQUE ANDRE L [US]) 27 December 2007 (2007-12-27) * the whole document * ----- | 1-12 | INV. A61H33/06 |
| A | US 2004/237179 A1 (KJONAAS ROGER [US]) 2 December 2004 (2004-12-02) * the whole document * ----- | 1-12 | |

**TECHNICAL FIELDS SEARCHED (IPC)**

A61H

The present search report has been drawn up for all claims

| Place of search | Date of completion of the search | Examiner |
|---|---|---|
| Munich | 21 October 2025 | Schindler-Bauer, P |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    ...........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 17 4504

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2025

| Patent document cited in search report | Publication date | Patent family member(s) | | Publication date |
|---|---|---|---|---|
| US 2007294819 A1 | 27-12-2007 | NONE | | |
| US 2004237179 A1 | 02-12-2004 | US | 2004237179 A1 | 02-12-2004 |
| | | US | 2009070926 A1 | 19-03-2009 |

EPO FORM P0459

For more details about this annex : see Official Journal of the European Patent Office, No. 12/82